# FASCICULE DE BREVET EUROPEEN

(11) **EP 2 486 088 B1**
(45) Date de publication et mention de la délivrance du brevet: **04.09.2013**
(21) Numéro de dépôt: 10768440.9
(22) Date de dépôt: 08.10.2010
(51) Int. Cl.: C08K 5/00, C08K 5/3492, B60C 1/00, C07D 253/02, C08K 5/43

(54) **COMPOSITION DE CAOUTCHOUC COMPRENANT UNE 1, 2, 4 - TRIAZINE**
KAUTSCHUKZUSAMMENSETZUNG MIT EINER 1,2,4-TRIAZINEVERBINDUNG
RUBBER COMPOSITION COMPRISING A 1,2,4-TRIAZINE DERIVATIVE COMPOUND

(30) Priorité: 08.10.2009 FR 0957037
(43) Date de publication de la demande: 15.08.2012
(73) Titulaire: COMPAGNIE GENERALE DES ETABLISSEMENTS MICHELIN, 63000 Clermont-Ferrand (FR); MICHELIN Recherche et Technique S.A., 1763 Granges-Paccot (CH)
(72) Inventeur: VEYLAND, Anne, F-63200 Marsat (FR); SEEBOTH, Nicolas, F-63000 Clermont-Ferrand (FR); ARAUJO DA SILVA, José Carlos, F-63430 Pont Du Chateau (FR)
(74) Mandataire: Sidhu, Alban
(86) Numéro de dépôt international: PCT/EP2010/065069
(87) Numéro de publication internationale: WO 2011/042522

(56) Documents cités:
- GB-A- 1 095 219
- US-A- 3 655 599

## Description

La présente invention se rapporte à une composition de caoutchouc utilisable notamment pour la fabrication de pneumatiques ou de produits semi-finis pour pneumatiques tels que des bandes de roulement, ladite composition étant à base d'un élastomère diénique, d'une charge renforçante et d'un système de vulcanisation comprenant un composé 1,2,4-triazine particulier.

La vulcanisation des élastomères diéniques par le soufre est largement utilisée dans l'industrie du caoutchouc, en particulier celle du pneumatique. Pour vulcaniser les élastomères diéniques, on utilise un système de vulcanisation relativement complexe comportant, en plus du soufre, un accélérateur primaire de vulcanisation, tels que les sulfénamides à noyau benzothiazole, ainsi que divers accélérateurs secondaires ou activateurs de vulcanisation, tout particulièrement des dérivés du zinc tels que l'oxyde de zinc (ZnO) seul ou utilisé avec des acides gras.

Les sulfénamides à noyau benzothiazole utilisés comme accélérateurs primaire de vulcanisation sont par exemple la N-cyclohexyl-2-benzothiazyle sulfénamide (en abrégé « CBS »), la N,N-dicyclohexyl-2-benzothiazyle sulfénamide (en abrégé « DCBS »), la N-ter-butyl-2-benzothiazyle sulfénamide (en abrégé « TBBS ») et les mélanges de ces composés.

Les accélérateurs de vulcanisation jouent un rôle important dans l'obtention d'une phase retard (délai d'induction), c'est-à-dire le temps nécessaire au début de la réaction de vulcanisation, et il est connu de l'homme du métier que ce paramétre est très difficile à ajuster. Il est donc particulièrement intéressant pour l'homme du métier d'avoir un accélérateur de vulcanisation induisant une phase retard longue, qu'il pourra ajuster s'il le souhaite par l'ajout d'accélérateurs complémentaires.

La demanderesse a découvert de nouvelles compositions de caoutchouc utilisables notamment pour la fabrication de pneumatiques, en particulier de bandes de roulement, à base d'élastomères diéniques, de charges renforçantes et d'un système de vulcanisation présentant une réticulation suffisante tout en ayant une phase retard plus longue, tout en conservant les propriétés habituelles des compositions de caoutchouc.

L'invention a donc pour objet une composition de caoutchouc pour la fabrication de pneumatiques, à base d'un ou plusieurs élastomères diéniques, d'une ou plusieurs charges renforçantes et d'un système de vulcanisation, ledit système de vulcanisation comprenant un ou plusieurs composés 1,2,4-triazines de formule 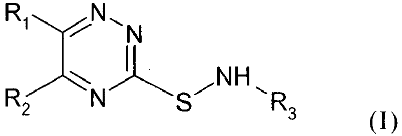 où
R₁ et R₂ représentent indépendamment H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes, R₁ et R₂ pouvant former ensemble un cycle,
R₃ représente :
- un groupe alkyle en C₁-C₂₅, linéaire ou ramifié, éventuellement interrompu par un ou plusieurs hétéroatomes, éventuellement substitué par un ou plusieurs groupes alkyles cycliques en C₃-C₁₀ ou aryles en C₆-C₁₂, ou
- un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

L'invention a également pour objet un procédé pour préparer une composition de caoutchouc pour la fabrication de pneumatiques telle que définie à l'une quelconque des revendications précédentes caractérisé en ce qu'il comprend les étapes suivantes :
- incorporer à ou aux élastomères diéniques, au cours d'une première étape dite « non-productive », la ou les charges renforçantes, en malaxant thermomécaniquement le tout, en une ou plusieurs fois, jusqu'à atteindre une température maximale comprise entre 130°C et 200°C,
- refroidir l'ensemble à une température inférieure à 100°C,
- incorporer ensuite, au cours d'une seconde étape dite « productive », le système de vulcanisation, puis
- malaxer le tout jusqu'à une température maximale inférieure à 120°C.

L'invention a encore pour objet l'utilisation d'une composition selon l'invention pour la fabrication d'un article fini ou d'un produit semi-fini destiné à un système de liaison au sol de véhicule automobile, tel que pneumatique, appui interne de sécurité pour pneumatique, roue, ressort en caoutchouc, articulation élastomérique, autre élément de suspension et anti-vibratoire. En particulier, la composition selon l'invention peut être utilisée pour la fabrication de produits semi-finis en caoutchouc destinés à des pneumatiques, tel que les bandes de roulement, les nappes d'armature de sommet, les flancs, les nappes d'armature de carcasse, les bourrelets, les protecteurs, les sous-couches, les blocs de caoutchouc et autres gommes internes, notamment les gommes de découplage, destinés à assurer la liaison ou l'interface entre les zones précitées des pneumatiques.

L'invention a encore pour objet un article fini ou produit semi-fini destiné à un système de liaison au sol de véhicule automobile, en particulier les pneumatiques et produits semi-finis pour pneumatiques, comprenant une composition selon l'invention. Les pneumatiques conformes à l'invention sont notamment destinés à des véhicules tourisme comme à des véhicules industriels choisis parmi camionnettes, « Poids-lourd »- i.e. métro, bus, engins de transport routier (camions, tracteurs, remorques), véhicules hors-la-route -, engin agricoles ou de génie civil, avions, autres véhicules de transport ou de manutention.

L'invention a enfin pour objet un l'utilisation comme accélérateur de vulcanisation dans une composition à base d'un ou plusieurs élastomères diéniques, d'une ou plusieurs charges renforçantes et d'un système de vulcanisation, d'un ou plusieurs composés 1,2,4-triazines de formule (1).

L'invention ainsi que ses avantages seront aisément compris à la lumière de la description et des exemples de réalisation qui suivent.

### 1. Mesures et tests utilisés

Les compositions de caoutchouc, dans lesquelles sont testés les accélérateurs de vulcanisation 1,2,4-triazines, sont caractérisées, avant et après cuisson, comme indiqué ci-après.

### Plasticité Mooney

On utilise un consistomètre oscillant tel que décrit dans la norme française NF T 43-005 (1991). La mesure de plasticité Mooney se fait selon le principe suivant : la composition à l'état cru (i.e., avant cuisson) est moulée dans une enceinte cylindrique chauffée à 100°C. Après une minute de préchauffage, le rotor tourne au sein de l'éprouvette à 2 tours/minute et on mesure le couple utile pour entretenir ce mouvement après 4 minutes de rotation. La plasticité Mooney (ML 1+4) est exprimée en "unité Mooney" (UM, avec 1 UM = 0,83 Newton.mètre).

### Rhéométrie

Les mesures sont effectuées à 150°C avec un rhéomètre à chambre oscillante, selon la norme DIN 53529 - partie 3 (juin 1983). L'évolution du couple rhéométrique, ΔCouple, en fonction du temps décrit l'évolution de la rigidification de la composition par suite de la réaction de vulcanisation. Les mesures sont traitées selon la norme DIN 53529 - partie 2 (mars 1983) : T₀ est le délai d'induction, c'est-à-dire le temps nécessaire au début de la réaction de vulcanisation ; T_{α} (par exemple T₉₉) est le temps nécessaire pour atteindre une conversion de α%, c'est-à-dire α% (par exemple 99%) de l'écart entre les couples minimum et maximum. On mesure également la constante de vitesse de conversion notée K (exprimée en min-1), d'ordre 1, calculée entre 30% et 80% de conversion, qui permet d'apprécier la cinétique de vulcanisation.

### Essais de traction

Ces essais de traction permettent de déterminer les contraintes d'élasticité et les propriétés à la rupture. Sauf indication différente, ils sont effectués conformément à la norme française NF T 46-002 de septembre 1988. On mesure en seconde élongation (i.e., après un cycle d'accommodation au taux d'extension prévu pour la mesure elle-même) le module sécant nominal (ou contrainte apparente, en MPa) à 10% d'allongement (notés MA10), à 100% d'allongement (notés MA100) et à 300% d'allongement (notés MA300).

Les contraintes à la rupture (en MPa) et les allongements à la rupture (en %) sont également mesurés. Toutes ces mesures de traction sont effectuées à la température de 100°C ± 2°C, et dans les conditions normales d'hygrométrie (50 ± 5% d'humidité relative), selon la norme française NF T 40-101 (décembre 1979).

### Propriétés dynamiques

Les propriétés dynamiques ΔG* et tan(δ)ₘₐₓ sont mesurées sur un viscoanalyseur (Metravib VA4000), selon la norme ASTM D 5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10Hz, dans les conditions normales de température (23°C) selon la norme ASTM D 1349-99, ou selon les cas à une température différente. On effectue un balayage en amplitude de déformation de 0,1% à 45% (cycle aller), puis de 45% à 0,1% (cycle retour). Les résultats exploités sont le module complexe de cisaillement dynamique (G*) et le facteur de perte tan(δ). Pour le cycle retour, on indique la valeur maximale de tan(δ) observée, noté tan(δ)ₘₐₓ, ainsi que l'écart de module complexe (ΔG*) entre les valeurs à 0,1% et à 45% de déformation (effet Payne).

### II. Conditions de réalisation de l'invention

Comme expliqué précédemment, la composition selon l'invention est à base d'un ou plusieurs élastomères diéniques, d'une ou plusieurs charges renforçantes et d'un système de vulcanisation.

Par l'expression composition « à base de », il faut entendre une composition comportant le mélange et/ou le produit de réaction des différents constituants utilisés, certains de ces constituants de base étant susceptibles de, ou destinés à réagir entre eux, au moins en partie, lors des différentes phases de fabrication de la composition, en particulier au cours de sa vulcanisation.

Dans la présente description, sauf indication expresse différente, tous les pourcentages (%) sont des % en masse. D'autre part, tout intervalle de valeurs désigné par l'expression "entre a et b" représente le domaine de valeurs allant de plus de a à moins de b (c'est-à-dire bornes a et b exclues) tandis que tout intervalle de valeurs désigné par l'expression "de a à b" signifie le domaine de valeurs allant de a jusqu'à b (c'est-à-dire incluant les bornes strictes a et b).

### II-1. Elastomère diénique

Par élastomère ou caoutchouc "diénique", doit être compris de manière connue un élastomère issu au moins en partie (i.e., un liomopolymère ou un copolymère) de monomères diènes (monomères porteurs de deux doubles liaisons carbone-carbone, conjuguées ou non).

Ces élastomères diénique peuvent être classés dans deux catégories : "essentiellement insaturés" ou "essentiellement saturés". On entend en général par "essentiellement insaturé", un élastomère diénique issu au moins en partie de monomères diènes conjugués, ayant un taux de motifs ou unités d'origine diénique (diènes conjugués) qui est supérieur à 15% (% en moles) ; c'est ainsi que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques "essentiellement saturés" (taux de motifs d'origine diénique faible ou très faible, toujours inférieur à 15%). Dans la catégorie des élastomères diéniques "essentiellement insaturés", on entend en particulier par élastomère diénique "fortement insaturé" un élastomère diénique ayant un taux de motifs d'origine diénique (diènes conjugués) qui est supérieur à 50%.

Ces définitions étant données, on entend plus particulièrement par élastomère diénique susceptible d'être utilisé dans les compositions conformes à l'invention:
(a) - tout homopolymère obtenu par polymérisation d'un monomère diène conjugué ayant de 4 à 12 atomes de carbone;
(b) - tout copolymère obtenu par copolymérisation d'un ou plusieurs diènes conjugués entre eux ou avec un ou plusieurs composés vinyle aromatique ayant de 8 à 20 atomes de carbone;
(c) - un copolymère ternaire obtenu par copolymérisation d'éthylène, d'une α-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène;
(d) - un copolymère d'isobutène et d'isoprène (caoutchouc butyle), ainsi que les versions halogénées, en particulier chlorées ou bromées, de ce type de copolymère.

Bien qu'elle s'applique à tout type d'élastomère diénique, l'homme du métier du pneumatique comprendra que la présente invention est de préférence mise en oeuvre avec des élastomères diéniques essentiellement insaturés, en particulier du type (a) ou (b) ci-dessus.

A titre de diènes conjugués conviennent notamment le butadiène-1,3, le 2-méthyl-1,3-butadiéne, les 2,3-di(alkyle en C₁-C₅)-1,3-butadiènes tels que par exemple le 2,3-diméthyl-1,3-butadiène, le 2,3-diéthyl-1,3-butadiène, le 2-méthyl-3-éthyl-1,3-butadiéne, le 2-méthyl-3-isopropyl-1,3-butadiène, un aryl-1,3-butadiène, le 1,3-pentadiène, le 2,4-hexadiène. A titre de composés vinylaromatique conviennent par exemple le styrène, l'ortho-, méta-, para-méthylstyrène, le mélange commercial "vinyle-toluène", le para-tertiobutylstyrène, les méthoxystyrènes, les chlorostyrènes, le vinylmésitylène, le divinylbenzène, le vinylnaphtalène.

Les copolymères peuvent contenir entre 99% et 20% en poids d'unités diéniques et entre 1% et 80% en poids d'unités vinylaromatique. Les élastomères peuvent avoir toute microstructure qui est fonction des conditions de polymérisation utilisées, notamment de la présence ou non d'un agent modifiant et/ou randomisant et des quantités d'agent modifiant et/ou randomisant employées. Les élastomères peuvent être par exemple à blocs, statistiques, séquencés, microséquencés, et être préparés en dispersion, en émulsion ou en solution ; ils peuvent être couplés et/ou étoilés ou encore fonctionnalisés avec un agent de couplage et/ou d'étoilage ou de fonctionnalisation. Pour un couplage à du noir de carbone, on peut citer par exemple des groupes fonctionnels comprenant une liaison C-Sn ou des groupes fonctionnels aminés tels que aminobenzophénone par exemple ; pour un couplage à une charge inorganique renforçante telle que silice, on peut citer par exemple des groupes fonctionnels silanol ou polysiloxane ayant une extrémité silanol (tels que décrits par exemple dans FR 2 740 778, US 6 013 718 ou WO 2008/141702), des groupes alkoxysilane (tels que décrits par exemple dans FR 2 765 882 ou US 5 977 238), des groupes carboxyliques (tels que décrits par exemple dans WO 01/92402 ou US 6 815 473, WO 2004/096865 ou US 2006/0089445) ou encore des groupes polyéthers (tels que décrits par exemple dans EP 1 127 909, US 6 503 973, WO 2009/000750 ou WO 2009/000752). Comme autres exemples d'élastomères fonctionnalisés, on peut citer également des élastomères (tels que SBR, BR, NR ou IR) du type époxydés.

Conviennent les polybutadiènes et en particulier ceux ayant une teneur (% molaire) en unités -1,2 comprise entre 4% et 80% ou ceux ayant une teneur (% molaire) en cis-1,4 supérieure à 80%, les polyisoprènes, les copolymères de butadiène-styrène et en particulier ceux ayant une Tg (température de transition vitreuse (Tg, mesurée selon ASTM D3418) comprise entre 0°C et - 70°C et plus particulièrement entre - 10°C et - 60°C, une teneur en styrène comprise entre 5% et 60% en poids et plus particulièrement entre 20% et 50%, une teneur (% molaire) en liaisons -1,2 de la partie butadiénique comprise entre 4% et 75%, une teneur (% molaire) en liaisons trans-1,4 comprise entre 10% et 80%, les copolymères de butadiènz-isoprène et notamment ceux ayant une teneur en isoprène comprise entre 5% et 90% en poids et une Tg de - 40°C à - 80°C, les copolymères isoprène-styrène et notamment ceux ayant une teneur en styrène comprise entre 5% et 50% en poids et une Tg comprise entre 5°C et - 50°C. Dans le cas des copolymères de butadiène-styrèneisoprène conviennent notamment ceux ayant une teneur en styrène comprise entre 5% et 50% en poids et plus particulièrement comprise entre 10% et 40%, une teneur en isoprène comprise entre 15% et 60%) en poids et plus particulièrement entre 20% et 50%, une teneur en butadiène comprise entre 5% et 50% en poids et plus particulièrement comprise entre 20% et 40%, une teneur (% molaire) en unités -1,2 de la partie butadiénique comprise entre 4% et 85%, une teneur (% molaire) en unités trans -1,4 de la partie butadiénique comprise entre 6% et 80%, une teneur (% molaire) en unités -1,2 plus -3,4 de la partie isoprénique comprise entre 5% et 70% et une teneur (% molaire) en unités trans -1,4 de la partie isoprénique comprise entre 10% et 50%, et plus généralement tout copolymère butadiène-styrène-isoprène ayant une Tg comprise entre - 5°C et - 70°C.

En résumé, le ou les élastomères diéniques de la composition selon l'invention sont choisis préférentiellement dans le groupe des élastomères diéniques fortement insaturés constitué par les polybutadiènes (en abrégé "BR"), les polyisoprènes (IR) de synthèse, le caoutchouc naturel (NR), les copolymères de butadiène, les copolymères d'isoprène et les mélanges de ces élastomères. De tels copolymères sont plus préférentiellement choisis dans le groupe constitué par les copolymères de butadiène-styrène (SBR), les copolymères d'isoprène-butadiène (BIR), les copolymères d'isoprène-styrène (SIR) et les copolymères d'isoprène-butadiène-styrène (SBIR).

Selon un mode de réalisation particulier, l'élastomère diénique est majoritairement (i.e., pour plus de 50 pce) un SBR, qu'il s'agisse d'un SBR préparé en émulsion ("ESBR") ou d'un SBR préparé en solution ("SSBR"), ou un coupage (mélange) SBR/BR, SBR/NR (ou SBR/IR), BR/NR (ou BR/IR), ou encore SBR/BR/NR (ou SBR/BR/IR). Dans le cas d'un élastomère SBR (ESBR ou SSBR), on utilise notamment un SBR ayant une teneur en styrène moyenne, par exemple comprise entre 20% et 35% en poids, ou une teneur en styrène élevée, par exemple de 35 à 45%, une teneur en liaisons vinyliques de la partie butadiénique comprise entre 15% et 70%), une teneur (% molaire) en liaisons trans-1,4 comprise entre 15% et 75% et une Tg comprise entre - 10°C et - 55°C ; un tel SBR peut être avantageusement utilisé en mélange avec un BR possédant de préférence plus de 90% (% molaire) de liaisons cis-1,4.

Selon un autre mode de réalisation particulier, l'élastomère diénique est majoritairement (pour plus de 50 pce) un élastomère isoprénique. C'est le cas en particulier lorsque les compositions de l'invention sont destinées à constituer, dans les pneumatiques, les matrices de caoutchouc de certaines bandes de roulement (par exemple pour véhicules industriels), de nappes d'armature de sommet (par exemple de nappes de travail, nappes de protection ou nappes de frettage), de nappes d'armature de carcasse, de flancs, de bourrelets, de protecteurs, de sous-couches, de blocs de caoutchouc et autres gommes internes assurant l'interface entre les zones précitées des pneumatiques.

Par "élastomère isoprénique", on entend de manière connue un homopolymère ou un copolymère d'isoprène, en d'autres termes un élastomère diénique choisi dans le groupe constitué par le caoutchouc naturel (NR), les polyisoprènes de synthèse (IR), les différents copolymères d'isoprène et les mélanges de ces élastomères. Parmi les copolymères d'isoprène, on citera en particulier les copolymères d'isobutène-isoprène (caoutchouc butyle - IIR), d'isoprène-styrène (SIR), d'isoprène-butadiène (BIR) ou d'isoprène-butadiène-styrène (SBIR). Cet élastomère isoprénique est de préférence du caoutchouc naturel ou un polyisoprène cis-1,4 de synthèse; parmi ces polyisoprènes de synthèse, sont utilisés de préférence des polyisoprènes ayant un taux (% molaire) de liaisons cis-1,4 supérieur à 90%, plus préférentiellement encore supérieur à 98%.

Selon un autre mode de réalisation particulier, notamment lorsqu'elle est destinée à un flanc de pneumatique, à une gomme intérieure étanche de pneumatique sans chambre (ou autre élément imperméable à l'air), la composition conforme à l'invention peut contenir au moins un élastomère diénique essentiellement saturé, en particulier au moins un copolymère EPDM ou un caoutchouc butyle (éventuellement chloré ou bromé), que ces copolymères soient utilisés seuls ou en mélange avec des élastomères diéniques fortement insaturés tels que cités précédemment, notamment NR ou IR, BR ou SBR.

Selon un autre mode préférentiel de réalisation de l'invention, la composition de caoutchouc comprend un coupage d'un (un ou plusieurs) élastomère diénique dit "à haute Tg" présentant une Tg comprise entre - 70°C et 0°C et d'un (un ou plusieurs) élastomère diénique dit "à basse Tg" comprise entre -110°C et -80°C, plus préférentiellement entre -105°C et -90°C. L'élastomère à haute Tg est choisi de préférence dans le groupe constitué par les S-SBR, les E-SBR, le caoutchouc naturel, les polyisoprènes de synthèse (présentant un taux (% molaire) d'enchaînements cis-1,4 de préférence supérieur à 95%), les BIR, les SIR, les SBIR, et les mélanges de ces élastomères. L'élastomère à basse Tg comprend de préférence des unités butadiène selon un taux (% molaire) au moins égal à 70% ; il consiste de préférence en un polybutadiène (BR) présentant un taux (% molaire) d'enchaînements cis-1,4 supérieur à 90%.

Selon un autre mode particulier de réalisation de l'invention, la composition de caoutchouc comprend par exemple de 30 à 100 pce, en particulier de 50 à 100 pce, d'un élastomère à haute Tg en coupage avec 0 à 70 pce, en particulier de 0 à 50 pce, d'un élastomère à basse Tg ; selon un autre exemple, elle comporte pour la totalité des 100 pce un ou plusieurs SBR préparé(s) en solution.

Selon un autre mode particulier de réalisation de l'invention, l'élastomère diénique de la composition selon l'invention comprend un coupage d'un BR (à titre d'élastomère basse Tg) présentant un taux (% molaire) d'enchaînements cis-1,4 supérieur à 90%), avec un ou plusieurs S-SBR ou E-SBR (à titre d'élastomère(s) haute Tg).

La composition selon l'invention peut contenir un seul élastomère diénique ou un mélange de plusieurs élastomères diéniques, le ou les élastomères diéniques pouvant être utilisés en association avec tout type d'élastomère synthétique autre que diénique, voire avec des polymères autres que des élastomères, par exemple des polymères thermoplastiques.

### II-2. Charge renforçante

On peut utiliser tout type de charge renforçante connue pour ses capacités à renforcer une composition de caoutchouc utilisable pour la fabrication de pneumatiques, par exemple une charge organique tel que du noir de carbone, une charge inorganique renforçante telle que de la silice, ou encore un coupage de ces deux types de charge, notamment un coupage de noir de carbone et de silice.

Comme noirs de carbone conviennent tous les noirs de carbone, notamment les noirs du type HAF, ISAF, SAF conventionnellement utilisés dans les pneumatiques (noirs dits de grade pneumatique). Parmi ces derniers, on citera plus particulièrement les noirs de carbone renforçants des séries 100, 200 ou 300 (grades ASTM), comme par exemple les noirs N115, N 134, N234, N326, N330, N339, N347, N375, ou encore, selon les applications visées, les noirs de séries plus élevées (par exemple N660, N683, N772). Les noirs de carbone pourraient être par exemple déjà incorporés à l'élastomère isoprénique sous la forme d'un masterbatch (voir par exemple demandes WO 97/36724 ou WO 99/16600).

Comme exemples de charges organiques autres que des noirs de carbone, on peut citer les charges organiques de polyvinylaromatique fonctionnalisé telles que décrites dans les demandes WO-A-2006/069792 et WO-A-2006/069793.

Par "charge inorganique renforçante", doit être entendu dans la présente demande, par définition, toute charge inorganique ou minérale (quelles que soient sa couleur et son origine (naturelle ou de synthèse), encore appelée charge "blanche", charge "claire" voire "charge non noire" ("non-black filler") par opposition au noir de carbone, capable de renforcer à elle seule, sans autre moyen qu'un agent de couplage intermédiaire, une composition de caoutchouc destinée à la fabrication de pneumatiques, en d'autres termes apte à remplacer, dans sa fonction de renforcement, un noir de carbone conventionnel de grade pneumatique ; une telle charge se caractérise généralement, de manière connue, par la présence de groupes hydroxylc (-OH) à sa surface.

L'état physique sous lequel se présente la charge inorganique renforçante est indifférent, que ce soit sous forme de poudre, de microperles, de granulés, de billes ou toute autre forme densifiée appropriée. Bien entendu on entend également par charge inorganique renforçante des mélanges de différentes charges inorganiques renforçantes, en particulier de charges siliceuses et/ou alumineuses hautement dispersibles telles que décrites ci-après.

Comme charges inorganiques renforçantes conviennent notamment des charges minérales du type siliceuse, en particulier de la silice (SiO₂), ou du type alumineuse, en particulier de l'alumine (Al₂O₃). La silice utilisée peut être toute silice renforçante connue de l'homme du métier, notamment toute silice précipitée ou pyrogénée présentant une surface BET ainsi qu'une surface spécifique CTAB toutes deux inférieures à 450 m²/g, de préférence de 30 à 400 m²/g. A titres de silices précipitées hautement dispersibles (dites "HDS"), on citera par exemple les silices Ultrasil 7000 et Ultrasil 7005 de la société Degussa, les silices Zeosil 1165MP, 1135MP et 1115MP de la société Rhodia, la silice Hi-Sil EZ150G de la société PPG, les silices Zeopol 8715, 8745 et 8755 de la Société Huber, les silices à haute surface spécifique telles que décrites dans la demande WO 03/16837.

Lorsque la composition selon l'invention est destinée à des bandes de roulement de pneumatique à faible résistance au roulement, la charge inorganique renforçante utilisée, en particulier s'il s'agit de silice, a de préférence une surface BET comprise entre 45 et 400 m²/g, plus préférentiellement comprise entre 60 et 300 m²/g.

De manière préférentielle, le taux de charge renforçante totale (noir de carbone et/ou charge inorganique renforçante telle que silice) est compris entre 20 et 200 pce, plus préférentiellement entre 30 et 150 pce, l'optimum étant de manière connue différent selon les applications particulières visées : le niveau de renforcement attendu sur un pneumatique vélo, par exemple, est bien sûr inférieur à celui exigé sur un pneumatique apte à rouler à grande vitesse de manière soutenue, par exemple un pneu moto, un pneu pour véhicule de tourisme ou pour véhicule utilitaire tel que Poids lourd.

Selon un mode de réalisation préférentiel de l'invention, on utilise une charge renforçante comportant entre 30 et 150 pce, plus préférentiellement entre 50 et 120 pce de charge inorganique, particulièrement de silice, et optionnellement du noir de carbone ; le noir de carbone, lorsqu'il est présent, est utilisé de préférence à un taux inférieur à 20 pce, plus préférentiellement inférieur à 10 pce (par exemple entre 0,1 et 10 pce).

Pour coupler la charge inorganique renforçante à l'élastomère diénique, on utilise de manière connue un agent de couplage (ou agent de liaison) au moins bifonctionnel destiné à assurer une connexion suffisante, de nature chimique et/ou physique, entre la charge inorganique (surface de ses particules) et l'élastomère diénique, en particulier des organosilanes ou des polyorganosiloxanes bifonctionnels.

On utilise notamment des silanes polysulfurés, dits "symétriques" ou "asymétriques" selon leur structure particulière, tels que décrits par exemple dans les demandes WO03/002648 (ou US 2005/016651) et WO03/002649 (ou US 2005/016650).

Conviennent en particulier, sans que la définition ci-après soit limitative, des silanes polysulfurés dits "symétriques" répondant à la formule générale (III) suivante:

(III) Z - A - Sₓ - A - Z ,

dans laquelle:
- x est un entier de 2 à 8 (de préférence de 2 à 5) ;
- A est un radical hydrocarboné divalent (de préférence des groupements alkylène en C₁-C₁₈ ou des groupements arylène en C₆-C₁₂, plus particulièrement des alkylènes en C₁-C₁₀, notamment en C₁-C₄, en particulier le propylène) ;
- Z répond à l'une des formules ci-après: 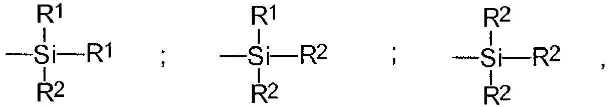 dans lesquelles:
- les radicaux R¹, substitués ou non substitués, identiques ou différents entre eux, représentent un groupe alkyle en C₁-C₁₈, cycloalkyle en C₅-C₁₈ ou aryle en C₆-C₁₈ (de préférence des groupes alkyle en C₁-C₆, cyclohexyle ou phényle, notamment des groupes alkyle en C₁-C₄, plus particulièrement le méthyle et/ou l'éthyle).
- les radicaux R², substitués ou non substitués, identiques ou différents entre eux, représentent un groupe alkoxyle en C₁-C₁₈ ou cycloalkoxyle en C₅-C₁₈ (de préférence un groupe choisi parmi alkoxyles en C₁-C₈ et cycloalkoxyles en C₅-C₈, plus préférentiellement encore un groupe choisi parmi alkoxyles en C₁-C₄, en particulier méthoxyle et éthoxyle).

Dans le cas d'un mélange d'alkoxysilanes polysulfurés répondant à la formule (III) ci-dessus, notamment des mélanges usuels disponibles commercialement, la valeur moyenne des "x" est un nombre fractionnaire de préférence compris entre 2 et 5, plus préférentiellement proche de 4. Mais l'invention peut être aussi avantageusement mise en oeuvre par exemple avec des alkoxysilanes disulfurés (x = 2).

A titre d'exemples de silanes polysulfurés, on citera plus particulièrement les polysulfures (notamment disulfures, trisulfures ou tétrasulfures) de bis-(alkoxyl(C₁-C₄)-alkyl(C₁-C₄)silyl-alkyl(C₁-C₄)), comme par exemple les polysulfures de bis(3-triméthoxysilylpropyl) ou de bis(3-triéthoxysilylpropyl). Parmi ces composés, on utilise en particulier le tétrasulfure de bis(3-triéthoxysilylpropyl), en abrégé TESPT, de formule [(C₂H₅O)₃Si(CH₂)₃S₂]₂ ou le disulfure de bis-(triéthoxysilylpropyle), en abrégé TESPD, de formule [(C₂H₅O)₃Si(CH₂);S]₂. On citera également à titre d'exemples préférentiels les polysulfures (notamment disulfures, trisulfures ou tétrasulfures) de bis-(monoalkoxyl(C₁-C₄)-dialkyl(C₁-C₄)silylpropyl), plus particulièrement le tétrasulfure de bis-monoéthoxydiméthylsilylpropyl tel que décrit dans la demande de brevet WO 02/083782 (ou US 2004/132880).

A titre d'agent de couplage autre qu'alkoxysilane polysulfuré, on citera notamment des POS (polyorganosiloxanes) bifonctionnels ou encore des polysulfures d'hydroxysilane (R² = OH dans la formule III ci-dessus) tels que décrits dans les demandes de brevet WO 02/30939 (ou US 6,774,255) et WO 02/31041 (ou US 2004/051210), ou encore des silanes ou POS porteurs de groupements fonctionnels azodicarbonyle, tels que décrits par exemple dans les demandes de brevet WO 2006/125532, WO 2006/125533, WO 2006/125534.

Dans les compositions de caoutchouc conformes à l'invention, la teneur en agent de couplage est préférentiellement comprise entre 4 et 12 pce, plus préférentiellement entre 3 et 8 pce.

L'homme du métier comprendra qu'à titre de charge équivalente de la charge inorganique renforçante décrite dans le présent paragraphe, pourrait être utilisée une charge renforçante d'une autre nature, notamment organique, dès lors que cette charge renforçante serait recouverte d'une couche inorganique telle que silice, ou bien comporterait à sa surface des sites fonctionnels, notamment hydroxyles, nécessitant l'utilisation d'un agent de couplage pour établir la liaison entre la charge et l'élastomère.

### 11.3 Système de vulcanisation

Le système de vulcanisation proprement dit est à base de soufre (ou d'un agent donneur de soufre) et d'un accélérateur primaire de vulcanisation. A ce système de vulcanisation de base viennent s'ajouter, incorporés au cours de la première phase non-productive et/ou au cours de la phase productive telles que décrites ultérieurement, divers accélérateurs secondaires ou activateurs de vulcanisation connus tels qu'oxyde de zinc, acide stéarique ou composés équivalents, dérivés guanidiques (en particulier diphénylguanidine).

Le soufre est utilisé à un taux préférentiel compris entre 0,5 et 10 pce, plus préférentiellement compris entre 0,5 et 5 pce, en particulier entre 0,5 et 3 pce lorsque la composition de l'invention est destinée, selon un mode préférentiel de l'invention, à constituer une bande de roulement de pneumatique.

L'accélérateur primaire de vulcanisation doit permettre une réticulation des compositions de caoutchouc dans des temps industriellement acceptables, tout en préservant un délai minimum de sécurité (« temps de grillage ») au cours duquel les compositions peuvent être mises en forme sans risque de vulcanisation prématurée (« grillage »).

Selon l'invention, le système de vulcanisation comprend, à titre d'accélérateur primaire de vulcanisation, un ou plusieurs composés 1,2,4-triazines de formule 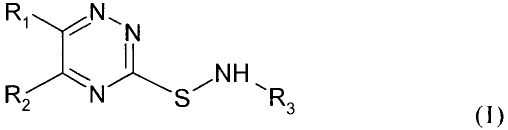 où
R₁ et R₂ représentent indépendamment H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes, R₁ et R₂ pouvant former ensemble un cycle,
R₃ représente :
- un groupe alkyle en C₁-C₂₅, linéaire ou ramifié, éventuellement interrompu par un ou plusieurs hétéroatomes, éventuellement substitué par un ou plusieurs groupes alkyles cycliques en C₃-C₁₀ ou aryles en C₆-C₁₂, ou
- un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

Les composés de formule (I) peuvent avantageusement remplacer en tout ou partie les composés sulfénamides classiquement utilisés.

Par groupe alkyle cyclique, on entend un groupe alkyle constitué d'un ou plusieurs cycles.

Le ou les hétéroatomes peuvent être un atome d'azote, de soufre ou d'oxygène.

R₁ et R₂ peuvent représenter indépendamment H, un groupe méthyle ou un groupe phényle.

Avantageusement, R₁ ou R₂ représentent un groupe phényle. De préférence, R₂ est un groupe phényle et R₁ est un hydrogène.

Avantageusement, R₃ représente un groupe cyclohexyle ou un groupe tertbutyle.

De préférence, R₃ représente un groupe cyclohexyle.

Ainsi, un composé de formule (I) préféré est celui dans lequel R₁ représente H, R₂ représente un groupe phényle et R₃ représente un cyclohexyle. Dans ce cas, le composé triazine de formule (1) est la 3-[(cyclohexylamino)thio]-5-phényl-1,2,4-triazine.

Selon un autre mode de réalisation préféré, R₃ représente un groupe tertbutyle.

Ainsi, un autre composé (I) préféré est celui dans lequel R₁ représente l'hydrogène, R₂ représente un groupe phényle et R₃ représente un groupe tertbutyle. Un tel composé est la 3-[(t-butylamino)thio]-5-phényl-1,2,4-triazine.

Le ou les composés de formule (I) représentent généralement de 0,1 à 10 pce, de préférence de 0,5 à 7 pce, mieux de 0,5 à 5 pce (parties en poids pour cent d'élastomère diénique).

Le ou les composés 1,2,4-triazines présents dans la composition selon l'invention peuvent être préparés selon le procédé comprenant les étapes suivantes :
- on part du composé (A) de formule suivante : 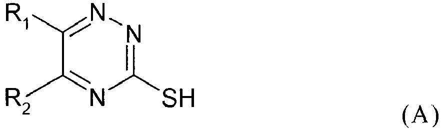 où R₁ et R₂ sont tels que définis précédemment, ce 1,2,4-triazinc-3-thiol pouvant être obtenu, sans que cela soit limitatif, par condensation entre un composé 1,2 dicarbonylé et le thiosemicarbazide selon des méthodes génériques décrites par exemple dans le Journal of Organic Chemistry, Vol 52, n° 19, 1987, p42R0-4287 ou encore par L. M. Mironovich et V.K. Promonenkov dans le chapitre intitulé 1,2,4-Triazines du volume 22 de l'ouvrage Bilans de la science et la technique. Série Chimie organique édité en 1990 par Viniti à Moscou ;
- on fait réagir le composé (A) avec une composition basique qui peut être une solution aqueuse d'une base organique ou minérale, par exemple une solution aqueuse d'hydroxyde de sodium, puis
- on ajoute au milieu réactionnel une amine primaire de formule R₃NH₂ ou R₃ est tel que défini précédemment, et on réalise un couplage oxydant en présence d'un composé oxydant qui peut être par exemple un composé halogéné tel que le dichlore, le dibrome, le diiode, les acides hypohalogéneux ou encore leurs sels alcalins tels que par exemple l'hypochlorite de sodium.

De préférence, R₁ représente l'hydrogène et R₂ représente un groupe phényle.

De préférence, R₃ représente un groupe cyclohexyle.

Le ou les composés 1,2,4-triazines de formule (I) présent dans la composition selon l'invention peuvent également être préparés selon un procédé de synthèse comportant les étapes suivantes :
a) on fait réagir de l'amine primaire de formule R₃NH₂, où R₃ est défini tel que précédemment, avec un composé oxydant, puis
b) on ajoute au milieu réactionnel obtenu à l'étape a) une composition comprenant une composition basique, de l'amine primaire de formule R₃NH₂, et un composé de formule (A) suivante : 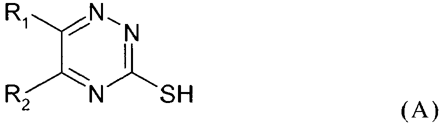
où R₁ et R₂ sont tels que définis précédemment,
ce composé pouvant être obtenu tel qu'indiqué précédemment.

De préférence, R₁ représente l'hydrogène et R₂ représente un groupe phényle.

De préférence, R₃ représente un groupe tertbutyle.

La composition basique peut être une solution aqueuse d'une base organique ou minérale, par exemple une solution aqueuse d'hydroxyde de sodium.

Le composé oxydant peut être choisi parmi les composés halogénés, de préférence le dichlore, le dibrome, le diiode et les acides hypohalogéneux et leurs sels alcalins. On peut citer en particulier l'hypochlorite de sodium.

Le système de vulcanisation de la composition selon l'invention peut également comprendre un ou plusieurs accélérateurs primaires additionnels, en particulier les composés de la famille des thiurames, les dérivés dithiocarbamates de zinc ou les thiophosphates.

### II-4. Additifs divers

La composition de caoutchouc selon l'invention peut comporter également tout ou partie des additifs usuels habituellement utilisés dans les compositions d'élastomères destinées à la fabrication de pneumatiques, en particulier de bandes de roulement, comme par exemple des plastifiants ou des huiles d'extension, que ces derniers soient de nature aromatique ou non-aromatique, des pigments, des agents de protection tels que cires anti-ozone (telle que la Cire Ozone C32 ST), anti-ozonants chimiques, anti-oxydants (tel que la 6-paraphénylènediamine), des agents anti-fatigue, des résines renforçantes, des accepteurs (par exemple résine phénolique novolaque) ou des donneurs de méthylène (par exemple HMT ou H3M) tels que décrits par exemple dans la demande WO 02/10269.

De préférence, la composition selon l'invention comporte, à titre d'agent plastifiant préférentiel non aromatique ou très faiblement aromatique, au moins un composé choisi dans le groupe constitué par les huiles naphténiques, paraffiniques, huiles MES, huiles TDAE, les esters (en particulier trioléates) de glycérol, les résines plastifiantes hydrocarbonées présentant une haute Tg de préférence supérieure à 30°C, et les mélanges de tels composés.

La composition selon l'invention peut également contenir, en complément des agents de couplage, des activateurs de couplage de la charge inorganique renforçante ou plus généralement des agents d'aide à la mise en oeuvre susceptibles de manière connue, grâce à une amélioration de la dispersion de la charge inorganique dans la matrice de caoutchouc et à un abaissement de la viscosité des compositions, d'améliorer leur faculté de mise en oeuvre à l'état cru, ces agents étant par exemple des silanes hydrolysables tels que des alkylalkoxysilanes (notamment des alkyltriéthoxysilanes), des polyols, des polyéthers (par exemple des polyéthylèneglycols), des amines primaires, secondaires ou tertiaires (par exemple des trialcanol-amines), des POS hydroxylés ou hydrolysables, par exemple des α,ω-dihydroxy-polyorganosiloxanes (notamment des α,ω-dihydroxy-polydiméthylsiloxanes), des acides gras comme par exemple l'acide stéarique.

### II-5. Fabrication des compositions de caoutchouc

La composition de caoutchouc selon l'invention est fabriquée dans des mélangeurs appropriés, en utilisant deux phases de préparation successives selon une procédure générale bien connue de l'homme du métier : une première phase de travail ou malaxage thermo-mécanique (parfois qualifiée de phase "non-productive") à haute température, jusqu'à une température maximale comprise entre 130°C et 200°C, de préférence entre 145°C et 185°C, suivie d'une seconde phase de travail mécanique (parfois qualifiée de phase "productive") à plus basse température, typiquement inférieure à 120°C, par exemple entre 60°C et 100°C, phase de finition au cours de laquelle est incorporé le système de réticulation ou vulcanisation.

Selon un mode de réalisation préférentiel de l'invention, tous les constituants de base de la composition de l'invention, à l'exception du système de vulcanisation, à savoir la ou les charges renforçantes, l'agent de couplage le cas échéant, sont incorporés de manière intime, par malaxage, à l'élastomère diénique ou aux élastomères diéniques au cours de la première phase dite non-productive, c'est-à-dire que l'on introduit dans le mélangeur et que l'on malaxe thermomécaniquement, en une ou plusieurs étapes, au moins ces différents constituants de base jusqu'à atteindre la température maximale comprise entre 130°C et 200°C, de préférence comprise entre 145°C et 185°C.

A titre d'exemple, la première phase (non-productive) est conduite en une seule étape thermomécanique au cours de laquelle on introduit, dans un mélangeur approprié tel qu'un mélangeur interne usuel, tous les constituants nécessaires, les éventuels agents de mise en oeuvre complémentaires et autres additifs divers, à l'exception du système de vulcanisation. La durée totale du malaxage, dans cette phase non-productive, est de préférence comprise entre 1 et 15 min. Après refroidissement du mélange ainsi obtenu au cours de la première phase non-productive, on incorpore alors le système de vulcanisation à basse température, généralement dans un mélangeur externe tel qu'un mélangeur à cylindres; le tout est alors mélangé (phase productive) pendant quelques minutes, par exemple entre 2 et 15 min.

La composition finale ainsi obtenue est ensuite calandrée par exemple sous la forme d'une feuille ou d'une plaque, notamment pour une caractérisation au laboratoire, ou encore extrudée sous la forme d'un profilé de caoutchouc utilisable par exemple comme une bande de roulement de pneumatique pour véhicule tourisme.

### III. Exemples de réalisation de l'invention

### III-1. La 3-[(t-butylamino)thio]-5-phényl-1,2,4-triazine sulfénamide

On décrit ici le procédé de préparation de la 3-[(t-butylamino)thio]-5-phényl-1,2,4-triazine sulfénamide (composé C) de formule suivante :

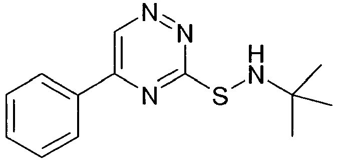

La préparation de cette sulfénamide repose sur un couplage oxydant entre le 5-phényl-1,2,4-triazine-3-thiol et la tertbutylamine, selon le schéma de synthèse suivant :

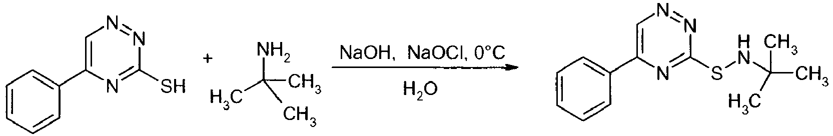

A 350 mL de tert-butylamine maintenue entre -10°C et -5 °C (température du bain), est ajoutée goutte à goutte une solution aqueuse de NaOCl (136 mL, titrée à 17,4 % de chlore actif) pendant 45 min. Pendant toute la durée d'addition la température du bain est maintenue entre -10°C et -5°C. A cette solution maintenue à 0°C, est ajouté goutte à goutte pendant 90 min une solution de 5-phényl-1,2,4-triazine-3-thiol (40,30 g, 0,213 mol; pureté : environ 98 % molaire. déterminée par RMN), d'hydroxyde de sodium (16,96 g, 0,424 mol) et de tert-butylamine (50 ml) dans l'eau (350 mL). La température du milieu réactionnel reste maintenue entre 0 et +6°C. Le milieu réactionnel est ensuite agité pendant une heure à température entre +5 et +10°C, puis 2 heures à température ambiante.

Ensuite on dilue ce milieu par 0,7 L d'eau froide (environ 4 °C). Cette suspension est encore agitée pendant 10 minutes puis le précipité du produit obtenu est filtré et lavé sur le filtre par de l'eau (10 fois 500 mL) puis séché 48 h sous air. Un solide jaune (49,5 g, 0,190 mol, rendement 89 %) de point de fusion 73 °C est obtenu. La pureté molaire déterminée par RMN ¹H est de 95%,

Le suivi de la réaction est réalisé par chromatographie sur couche mince :
Rf_{produit} = 0,52, Rf_{impureté disulfure} = 0,61 (caractéristiques de la CCM : SiO₂; EtOAc : heptane =1:1; révélation par UV et I₂).

La caractérisation complète du produit est réalisée par RMN. Les déplacements chimiques obtenus par RMN ¹H et ¹³C dans le DMSO-d6 sont donnés dans le tableau ci-dessous. La calibration est réalisée sur le DMSO (2,44 ppm en ¹H et à 39,5 ppm en ¹³C).

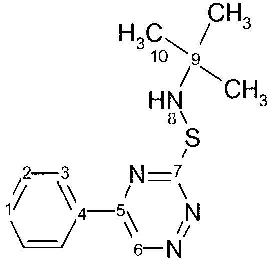

Les résultats sont donnés dans le tableau 1.

**Tableau 1**

| N° | 1 | 2 | 3 | 4 | 5 | 6 | 7 | 8 | 9 | 10 |
|---|---|---|---|---|---|---|---|---|---|---|
| δ ¹H ppm | 7,59 | 7,56 | 8,29 | - | - | 9,72 | - | 4,87 | - | 1,11 |
| δ ¹³C ppm | 132,7 | 128,9 | 127,7 | 129,7 | 153,7 | 142,9 | 177,0 | - | 54,3 | 29,1 |

### III-2. La 3-[(cyclohexylamino)thio]-5-phényl-1,2,4-triazine

Dans les exemples qui suivent, l'invention est mise en oeuvre avec la 3-[(cyclohexylamino)thio]-5-phényl-1,2,4-triazine (composé B) de formule suivante :

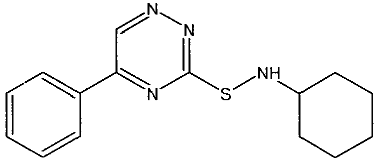

### III-2.1. Synthèse du composé triazine B

La préparation de ce composé est effectuée à partir du 5-phényl-1,2,4-triazine-3-thiol et de la cyclohexylamine, selon le schéma de synthèse suivant :

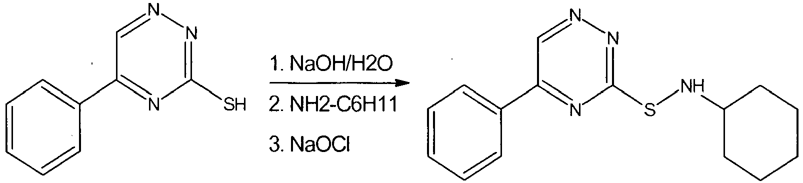

Le 5-phényl-1,2,4-triazine-3-thiol (numéro CAS [15969-28-5] est commercial et peut être obtenu selon des procédures décrites dans les documents suivants :
1. Daunis, J. et al.; Bulletin de la Société Chimique de France; 1969, 10; 3675 - 3678.
2. Joshi, Krishna C.; Dubey, Kalpana; Dandia, Anshu, Heterocycles, 198116; 9; 1545 - 1553.

A une solution de 5-phényl-1,2,4-triazine-3-thiol (41,0 g, 0,22 mol) et d'hydroxyde de sodium (20,0 g, 0,50 mol) dans H₂O (700 mL) est ajouté la cyclohexylamine (107,6 g, 1,09 mol). Le mélange est refroidi jusqu'à une température comprise entre 0 et -5°C puis est ajoutée goutte à goutte la solution aqueuse de NaOCl (4 % de chlore actif) (477 mL) pendant 30 minutes. La température du milieu réactionnel reste comprise entre 0 et - 4°C. Le milieu réactionnel est ensuite agité pendant 1h30 à 2 heures à une température comprise entre 0 et 5°C.

De l'éther de pétrole (100 mL, coupe 40/60°C) est ajouté et le milieu réactionnel est ensuite agité pendant 15 à 30 minutes à une température comprise entre 0 et -4°C. Le précipité est filtré et lavé sur le filtre par de l'eau (200 mL) et l'éther de pétrole (50 mL, coupe 40/60°C) et enfin séché pendant 2 à 3 heures sous pression réduite.

Un solide blanc (42,9 g, 0,15 mol, rendement 68 %) de point de fusion 125-127 °C est obtenu.

La pureté molaire est supérieure à 97 % (RMN ¹H).

Si la pureté n'est pas suffisante, une cristallisation dans l'acétate d'éthyle permet d'obtenir le produit pur.

La caractérisation complète du produit est réalisée par RMN. Les déplacements chimiques obtenus par RMN ¹H et ¹³C dans le DMSO-d6 sont donnés dans le tableau ci-dessous. La calibration est réalisée sur le DMSO (2,44 ppm en ¹H et à 39,5 ppm en ¹³C).

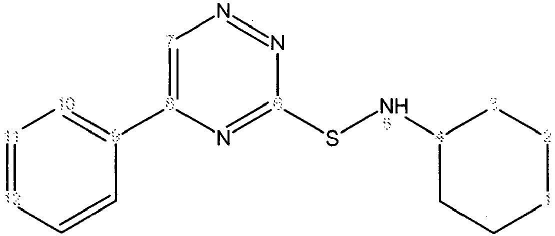

Les résultats sont indiqués dans le tableau 2.

**Tableau 2**

| N° | 1 | 2 | 3 | 4 | 5 | 6 | 7 | 8 | 9 | 10 | 11 | 12 |
|---|---|---|---|---|---|---|---|---|---|---|---|---|
| δ*(¹H) En ppm* | 1.45 | 1.63 | 1.92 | 2.97 | 5.08 | / | 9.73 | / | / | 8.27 | 7.56 | 7.6 |
| | 1.13 | 1.13 | 1.13 | | | | | | | | | |
| δ*(¹³C) En ppm* | 25.5 | 23.8 | 32.4 | 56,7 | / | 176.4 | 143.2 | 154.2 | 132.9 | 127.8 | 129.3 | 132.8 |

### III-2.2. Préparation des compositions

On procède pour les essais qui suivent de la manière suivante: on introduit dans un mélangeur interne, rempli à 70% et dont la température initiale de cuve est d'environ 90°C, le ou les élastomères diéniques, la ou les charges renforçantes, l'agent de couplage éventuel puis, après une à deux minutes de malaxage, les divers autres ingrédients à l'exception du système de vulcanisation. On conduit alors un travail thermomécanique (phase non-productive) en une étape (durée totale du malaxage égale à environ 5 min), jusqu'à atteindre une température maximale de "tombée" d'environ 165°C. On récupère le mélange ainsi obtenu, on le refroidit puis on ajoute le système de vulcanisation (soufre et composé 1,2,4-triazine) sur un mélangeur externe (homo-finisseur) à 70°C, en mélangeant le tout (phase productive) pendant environ 5 à 6 min.

Les compositions ainsi obtenues sont ensuite calandrées soit sous la forme de plaques (épaisseur de 2 à 3 mm) ou de feuilles fines de caoutchouc pour la mesure de leurs propriétés physiques ou mécaniques, soit sous la forme de profilés utilisables directement, après découpage et/ou assemblage aux dimensions souhaitées, par exemple comme produits semi-finis pour pneumatiques, en particulier comme bandes de roulement de pneumatiques.

### III-2.3. Essais de caractérisation - Résultats

### A- Exemple 1

L'objet de cet exemple est de comparer les propriétés d'une composition de caoutchouc comprenant du noir de carbone à titre de charge renforçante majoritaire, utilisable pour la fabrication d'une bande de roulement de pneumatique, comprenant la 3-[(cyclohexylamino)thio]-5-phényl-1,2,4-triazine (composé B) à titre d'accélérateur primaire de vulcanisation (composition 2) avec les propriétés d'une composition de caoutchouc comprenant la N-cyclohexyl-2-benzothiazyle sulfénamide (« CBS ») (composition 1).

Les formulations des compositions sont données dans le tableau 3. Les quantités sont exprimées en parties pour 100 parties en poids d'élastomère (pce).

**Tableau 3**

| | Composition 1 | Composition 2 |
|---|---|---|
| NR (1) | 40 | 40 |
| BR (2) | 20 | 20 |
| SBR (3) | 40 | 40 |
| N234 (4) | 54 | 54 |
| Paraffine | 1 | 1 |
| 6-PPD (5) | 2 | 2 |
| Acide stéarique | 2 | 2 |
| ZnO | 2,7 | 2,7 |
| Soufre | 1,1 | 1,1 |
| Accélérateur de vulcanisation | 1,1 * | 1,23 ** |

| | | |
|---|---|---|
| * CBS (« Santocure CBS » de la société Flexsys) ** 3-[(cyclohexylamino)thio]-5-phényl-1,2,4-triazine (1) caoutchouc naturel (2) polybutadiène avec 0,7% de 1-2 ; 1,7% de trans 1-4 ; 98% de cis 1-4 (Tg = -105°C) (% molaires) (3) copolymère butadiènc-styrène SSBR (SBR préparé en solution) avec 25% de styrène, 59% de motifs polybutadiène 1-2 et 20% de motifs polybutadiène 1-4 trans (Tg = -24°C) (% molaires) ; taux exprimé en SBR sec (SBR étendu avec 9% en poids d'huile MES, soit un total de SSBR + huile égal à 76 pce) (4) noir de carbone (5) Agent anti-oxydant 6-para-phénylènediamine | | |

La composition de caoutchouc 2 comprenant la 3-[(cyclohexylamino)thio]-5-phényl-1,2,4-triazine est identique à la composition 1, étant entendu que la CBS est remplacée par une quantité isomolaire de 3-[(cyclohexylamino)thio]-5-phényl-1,2,4-triazine.

Les propriétés rhéométriques à 150°C sont données dans le tableau 4.

**Tableau 4**

| | Composition 1 (CBS) | Composition 2 (composé B) |
|---|---|---|
| Prop.rhéo. *(DIN)* | 150°C | |
| Δcouple(dN.m) | 8,5 | 8,7 |
| k(min⁻¹) | 0,416 | 0,353 |
| t₀(min) | 6,0 | 8,6 |

Les propriétés rhéométriques obtenues pour la composition 2 contenant la 3-[(cyclohexylamino)thio]-5-phényl-1,2,4-triazine sont équivalentes à celles obtenues pour la composition 1 contenant la CBS. On note même que la phase retard (délai d'induction t₀) est plus long dans le cas de la composition 2 contenant la 3-[(cyclohexylamino)thio]-5-phényl-1,2,4-triazine, ce qui est avantageux.

On note, par ailleurs que le composé B, ainsi que les composés de formule (I) en général, remplacent avantageusement vis-à-vis de l'impact environnemental, les sulfénamides à noyau mercaptobenzothiazole, en ne générant pas contrairement à ces derniers, de mercaptobenzothiazole en se décomposant au cours de la cuisson.

### B-Exemple 2

L'objet de cet exemple est de comparer les propriétés d'une composition de caoutchouc comprenant de la silice à titre de charge renforçante majoritaire, utilisable pour la fabrication d'une bande de roulement de pneumatique, comprenant la 3-[(cyclohexylamino)thio]-5-phényl-1,2,4-triazine (composé B) à titre d'accélérateur primaire de vulcanisation (composition 4) avec les propriétés d'une composition de caoutchouc comprenant la N-cyclohcxyl-2-bcnzothiazyle sulfénamide (« CBS ») (composition 3).

Les formulations des compositions sont données dans le tableau 5. Les quantités sont exprimées en parties pour 100 parties en poids d'élastomère (pce).

**Tableau 5**

| | Composition 3 | Composition 4 |
|---|---|---|
| BR (1) | 28 | 28 |
| SBR (2) | 79,2 | 79,2 |
| N234 (3) | 4 | 4 |
| Silice (4) | 82 | 82 |
| 6-PPD (5) | 1,9 | 1,9 |
| MES/HPD (6) | 4,8 | 4,8 |
| Cire ozone C32 ST (7) | 1,5 | 1,5 |
| Résine (8) | 20 | 20 |
| Silane liquide (9) | 6,56 | 6,56 |
| Acide stéarique | 2 | 2 |
| DPG (10) | 1,54 | 1,54 |
| ZnO | 1,5 | 1,5 |
| Soufre | 1,2 | 1,2 |
| Accélérateur de vulcanisation | 1,9* | 2,12** |

| | | |
|---|---|---|
| * CBS (« Santocure CBS » de la société Flexsys) ** 3-[(cyclohexylamino)thio]-5-phényl-1,2,4-triazine (1) polybutadiène avec 0,7% de 1-2 ; 1,7% de trans 1-4 ; 98% de cis 1-4 (Tg = -105°C) (% molaires) (2) copolymère butadiène-styrène SSBR (SBR préparé en solution) avec 25% de styrène, 59% de motifs polybutadiène 1-2 et 20% de motifs polybutadiène 1-4 trans (Tg = -24°C) (% molaires) ; taux exprimé en SBR sec (SBR étendu avec 9% en poids d'huile MES, soit un total de SSBR + huile égal à 76 pce) (3) noir de carbone (4) Silice "Zeosil 1 165MP" de la société Rhodia, type "HDS" (BET et CTAB : environ 160 m²/g); (5) Agent anti-oxydant 6-para-phénylènediamine (6) Huile plastifiante « Medium Extracted Solvates » (Catenex SNR de Shell) (7) Agent anti-ozonant (8) Résine aliphatique (pure C5) « Hikorez A-1100 » commercialisée par la société KOLON (9) agent de couplage (10) Diphénylguanidine (Perkacit DPG de la société Flexsys) | | |

La composition de caoutchouc 4 comprenant la 3-[(cyclohexylamino)thio]-5-phényl-1,2,4-triazine est identique à la composition 3, étant entendu que la CBS est remplacée par une quantité isomolaire de 3-[(cyclohexylamino)thio]-5-phényl-1,2,4-triazine.

Les propriétés rhéométriques à 150°C sont données dans le tableau 6.

**Tableau 6**

| | Composition 3 (CBS) | Composition 4 (composé B) |
|---|---|---|
| Prop.rhéo. *(DIN)* | 150°C | |
| Δcouple(dN.m) | 11,9 | 12,2 |
| k(min⁻¹) | 0,064 | 0,052 |
| t₀(min) | 4,9 | 6,9 |

Les propriétés rhéométriques obtenues pour la composition 4 comprenant la 3-[(cyclohexylamino)thio]-5-phényl-1,2,4-triazine sont équivalentes à celles obtenues pour la composition 3 contenant la CBS. On note même que la phase retard (délai d'induction t₀) est plus long dans le cas de la composition contenant la 3-[(cyclohexylamino)thio]-5-phényl-1,2,4-triazine, ce qui est avantageux.

Des résultats comparables peuvent être obtenus avec la 3-[(t-butylamino)thio]-5-phényl-1,2,4-triazine sulfénamide.

En conclusion, les composés 1,2,4-triazines de l'invention utilisés comme accélérateur de vulcanisation dans des compositions de caoutchouc comprenant une ou des charges renforçantes permettent d'améliorer la phase retard (délai d'induction t₀).

## Revendications

1. Composition de caoutchouc pour la fabrication de pneumatiques, à base d'un ou plusieurs élastomères diéniques, d'une ou plusieurs charges renforçantes et d'un système de vulcanisation, **caractérisée en ce que** ledit système de vulcanisation comprend un ou plusieurs composés 1,2,4-triazines de formule 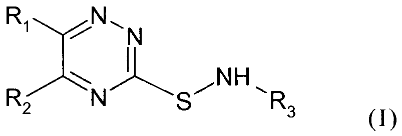 où
R₁ et R₂ représentent indépendamment H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes, R₁ et R₂ pouvant former ensemble un cycle,
R₃ représente :
- un groupe alkyle en C₁-C₂₅, linéaire ou ramifié, éventuellement interrompu par un ou plusieurs hétéroatomes, éventuellement substitué par un ou plusieurs groupes alkyles cycliques en C₃-C₁₀ ou aryles en C₆-C₁₂, ou
- un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

2. Composition selon la revendication 1 **caractérisée en ce que** R₁ et R₂ représentent indépendamment H, un groupe méthyle ou un groupe phényle.

3. Composition selon la revendication 2 **caractérisée en ce que** R₁ ou R₂ représentent un groupe phényle.

4. Composition selon la revendication 3 **caractérisée en ce que** R₁ représente un hydrogène et R₂ représentent un groupe phényle.

5. Composition selon l'une quelconque des revendications précédentes **caractérisée en ce que** R₃ représente un groupe cyclohexyle ou un groupe tertbutyle.

6. Composition selon l'une quelconque des revendications précédentes **caractérisée en ce que** le ou lesdits composés 1,2,4-triazines représentent de 0,1 à 10 pce, de préférence de 0,5 à 7 pee, mieux de 0,5 à 5 (parties en poids pour cent d'élastomère diénique).

7. Composition selon l'une quelconque des revendications précédentes **caractérisée en ce que** le ou les élastomères diéniques sont choisis dans le groupe constitué par les polybutadiènes, le caoutchouc naturel, les polyisoprènes de synthèse, les copolymères de butadiène, les copolymères d'isoprène et les mélanges de ces élastomères.

8. Composition selon la revendication 7 **caractérisée en ce que** l'élastomère diénique est un coupage de caoutchouc naturel avec un polybutadiène et un copolymère de butadiène-styrène, ou un coupage d'un polybutadiène et d'un copolymère de butadiène-styrène.

9. Composition selon l'une quelconque des revendications précédentes **caractérisée en ce que** la ou les charges renforçantes sont choisies parmi la silice, le noir de carbone et leurs mélanges.

10. Composition selon l'une quelconque des revendications précédentes **caractérisée en ce que** la ou les charges renforçantes sont présentes à un taux compris entre 20 et 200 pce, de préférence entre 30 et 150 pce.

11. Procédé pour préparer une composition de caoutchouc pour la fabrication de pneumatiques telle que définie à l'une quelconque des revendications précédentes **caractérisé en ce qu'**il comprend les étapes suivantes :
- incorporer à ou aux élastomères diéniques, au cours d'une première étape dite « non-productive », la ou les charges renforçantes, en malaxant thermomécaniquement le tout, en une ou plusieurs fois, jusqu'à atteindre une température maximale comprise entre 130°C et 200°C,
- refroidir l'ensemble à une température inférieure à 100°C,
- incorporer ensuite, au cours d'une seconde étape dite « productive », le système de vulcanisation, puis
- malaxer le tout jusqu'à une température maximale inférieure à 120°C.

12. Utilisation d'une composition selon l'une quelconque des revendications 1 à 10, pour la fabrication d'un article fini ou d'un produit semi-fini destiné à un système de liaison au sol de véhicule automobile.

13. Article fini ou produit semi-fini destiné à un système de liaison au sol de véhicule automobile, comprenant une composition selon l'une quelconque des revendications 1 à 10.

14. Pneumatique comprenant une composition de caoutchouc telle que définie à l'une quelconque des revendications 1 à 10.

15. Utilisation comme accélérateur de vulcanisation dans une composition à base d'un ou plusieurs élastomères diéniques, d'une ou plusieurs charges renforçantes et d'un système de vulcanisation, d'un ou plusieurs composés 1,2,4-triazines de formule (I) tels que définis dans l'une quelconque des revendications 1 à 5.

## Patentansprüche

1. Kautschukzusammensetzung zur Herstellung von Reifen auf der Basis eines oder mehrerer Dienelastomere, eines oder mehrerer verstärkender Füllstoffe und eines Vulkanisationssystems, **dadurch gekennzeichnet, dass** das Vulkanisationssystem eine oder mehrere 1,2,4-Triazinverbindungen der Formel 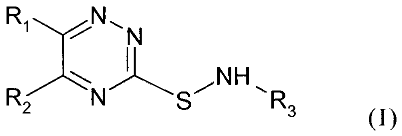 umfasst, wobei
R₁ und R₂ unabhängig für H oder eine C₁-C₂₅-Kohlenwasserstoffgruppe, die aus linearen, verzweigten oder cyclischen Alkylgruppen und Arylgruppen, die gegebenenfalls durch ein oder mehrere Heteroatome unterbrochen sind, ausgewählt ist, stehen, wobei R₁ und R₂ zusammen einen Ring bilden können,
R₃ für:
- eine lineare oder verzweigte C₁-C₂₅-Alkylgruppe, die gegebenenfalls durch ein oder mehrere Hetero-atome unterbrochen ist und gegebenenfalls durch eine oder mehrere C₃-C₁₀-Cycloalkyl- oder C₆-C₁₂-Arylgruppen substituiert ist, oder
- eine C₃-C₁₀-Cycloalkylgruppe, die gegebenenfalls durch einen oder mehrere Heteroatome unterbrochen ist und gegebenenfalls durch eine oder mehrere lineare oder verzweigte oder cyclische C₁-C₂₅-Alkyl- oder C₆-C₁₂-Arylgruppen, die gegebenenfalls durch ein oder mehrere Heteroatome unterbrochen sind, substituiert ist,
steht.

2. Zusammensetzung nach Anspruch 1, **dadurch gekennzeichnet, dass** R₁ und R₂ unabhängig für H, eine Methylgruppe oder eine Phenylgruppe stehen.

3. Zusammensetzung nach Anspruch 2, **dadurch gekennzeichnet, dass** R₁ und R₂ für eine Phenylgruppe stehen.

4. Zusammensetzung nach Anspruch 3, **dadurch gekennzeichnet, dass** R₁ für Wasserstoff steht und R₂ für eine Phenylgruppe steht.

5. Zusammensetzung nach einem der vorhergehenden Ansprüche, **dadurch gekennzeichnet, dass** R₃ für eine Cyclohexylgruppe oder eine tert.-Butylgruppe steht.

6. Zusammensetzung nach einem der vorhergehenden Ansprüche, **dadurch gekennzeichnet, dass** die 1,2,4-Triazinverbindung bzw. die 1,2,4-Triazinverbindungen 0,1 bis 10 phe, vorzugsweise 0,5 bis 7 phe und noch besser 0,5 bis 5 phe (Gewichtsteile pro hundert Teile Dienelastomer) ausmacht bzw. ausmachen.

7. Zusammensetzung nach einem der vorhergehenden Ansprüche, **dadurch gekennzeichnet, dass** das Dienelastomer bzw. die Dienelastomere aus der Gruppe bestehend aus Polybutadienen, Naturkautschuk, synthetischen Polyisoprenen, Butadien-Copolymeren, Isopren-Copolymeren und Mischungen dieser Elastomere ausgewählt ist bzw. sind.

8. Zusammensetzung nach Anspruch 7, **dadurch gekennzeichnet, dass** es sich bei dem Dienelastomer um eine Mischung von Naturkautschuk mit einem Polybutadien und einem Butadien-Styrol-Copolymer oder eine Mischung eines Polybutadiens und eines Butadien-Styrol-Copolymers handelt.

9. Zusammensetzung nach einem der vorhergehenden Ansprüche, **dadurch gekennzeichnet, dass** der verstärkende Füllstoff bzw. die verstärkenden Füllstoffe aus Kieselsäure, Ruß und Mischungen davon ausgewählt ist bzw. sind.

10. Zusammensetzung nach einem der vorhergehenden Ansprüche, **dadurch gekennzeichnet, dass** der verstärkende Füllstoff bzw. die verstärkenden Füllstoffe in einer Menge zwischen 20 und 200 phe und vorzugsweise zwischen 30 und 150 phe vorliegt bzw. vorliegen.

11. Verfahren zur Herstellung einer Kautschukzusammensetzung zur Herstellung von Reifen gemäß einem der vorhergehenden Ansprüche, **dadurch gekennzeichnet, dass** man:
- im Lauf eines ersten, als "nichtproduktiv" bezeichneten Schritts den verstärkenden Füllstoff bzw. die verstärkenden Füllstoffe in das Dienelastomer bzw. die Dienelastomere einarbeitet, indem man das Ganze ein- oder mehrmals thermomechanisch knetet, bis eine Höchsttemperatur zwischen 130°C und 200°C erreicht ist;
- die Masse auf eine Temperatur unter 100°C abkühlt;
- dann im Lauf eines zweiten, als "produktiv" bezeichneten Schritts das Vulkanisationssystem einarbeitet und dann
- das Ganze bis zu einer Höchsttemperatur von weniger als 120°C knetet.

12. Verwendung einer Zusammensetzung nach einem der Ansprüche 1 bis 10 zur Herstellung eines Fertigartikels oder Halbzeugs für ein Bodenkontaktsystem eines Kraftfahrzeugs.

13. Fertigartikel oder Halbzeug für ein Bodenkontaktsystem eines Kraftfahrzeugs, umfassend eine Zusammensetzung nach einem der Ansprüche 1 bis 10.

14. Reifen, umfassend eine Kautschukzusammensetzung gemäß einem der Ansprüche 1 bis 10.

15. Verwendung einer oder mehrerer 1,2,4-Triazinverbindungen der Formel (I) gemäß einem der Ansprüche 1 bis 5 als Vulkanisationsbeschleuniger in einer Zusammensetzung auf der Basis eines oder mehrerer Dienelastomere, eines oder mehrerer verstärkender Füllstoffe und eines Vulkanisationssystems.

## Claims

1. Rubber composition for the manufacture of tyres, based on one or more diene elastomers, on one or more reinforcing fillers and on a vulcanization system, **characterized in that** the said vulcanization system comprises one or more 1,2,4-triazine compounds of formula: 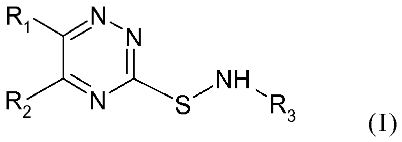 where
R₁ and R₂ independently represent H or a C₁-C₂₅-hydrocarbon group chosen from linear, branched or cyclic alkyl groups and aryl groups, optionally interrupted by one or more heteroatoms, it being possible for R₁ and R₂ to together form a ring,
R₃ represents:
- a linear or branched C₁-C₂₅ alkyl group which is optionally interrupted by one or more heteroatoms and which is optionally substituted by one or more cyclic C₃-C₁₀ alkyl or C₆-C₁₂ aryl groups, or
- a cyclic C₃-C₁₀ alkyl group which is optionally interrupted by one or more heteroatoms and which is optionally substituted by one or more linear, branched or cyclic C₁-C₂₅ alkyl or C₆-C₁₂ aryl groups which are optionally interrupted by one or more heteroatoms.

2. Composition according to Claim 1, **characterized in that** R₁ and R₂ independently represent H, a methyl group or a phenyl group.

3. Composition according to Claim 2, **characterized in that** R₁ or R₂ represents a phenyl group.

4. Composition according to Claim 3, **characterized in that** R₁ represents a hydrogen and R₂ represents a phenyl group.

5. Composition according to any one of the preceding claims, **characterized in that** R₃ represents a cyclohexyl group or a tert-butyl group.

6. Composition according to any one of the preceding claims, **characterized in that** the said 1,2,4-triazine compound or compounds represent from 0.1 to 10 phr, preferably from 0.5 to 7 phr and better still from 0.5 to 5 phr (parts by weight per hundred of diene elastomer).

7. Composition according to any one of the preceding claims, **characterized in that** the diene elastomer or elastomers are chosen from the group consisting of polybutadienes, natural rubber, synthetic polyisoprenes, butadiene copolymers, isoprene copolymers and the mixtures of these elastomers.

8. Composition according to Claim 7, **characterized in that** the diene elastomer is a blend of natural rubber with a polybutadiene and a butadiene/styrene copolymer, or a blend of a polybutadiene and a butadiene/styrene copolymer.

9. Composition according to any one of the preceding claims, **characterized in that** the reinforcing filler or fillers are chosen from silica, carbon black and their mixtures.

10. Composition according to any one of the preceding claims, **characterized in that** the reinforcing filler or fillers are present at a level of between 20 and 200 phr, preferably between 30 and 150 phr.

11. Process for preparing a rubber composition for the manufacture of tyres as defined in any one of the preceding claims, **characterized in that** it comprises the following stages:
- incorporating the reinforcing filler or fillers in the diene elastomer or elastomers, during a first "non-productive" stage, everything being kneaded thermomechanically, in one or more goes, until a maximum temperature of between 130°C and 200°C is reached,
- cooling the combined mixture to a temperature of less than 100°C,
- subsequently incorporating, during a second "productive" stage, the vulcanization system and then
- kneading everything up to a maximum temperature of less than 120°C.

12. Use of a composition according to any one of Claims 1 to 10 in the manufacture of a finished article or a semi-finished product intended for a motor vehicle ground-contact system.

13. Finished article or semi-finished product intended for a motor vehicle ground-contact system, comprising a composition according to any one of Claims 1 to 10.

14. Tyre, comprising a rubber composition as defined in any one of Claims 1 to 10.

15. Use as vulcanization accelerator, in a composition based on one or more diene elastomers, on one or more reinforcing fillers and on a vulcanization system, of one or more 1,2,4-triazine compounds of formula (I) as defined in any one of Claims 1 to 5.
